# EUROPEAN PATENT SPECIFICATION

(11) **EP 3 550 299 B1**
(45) Date of publication and mention of the grant of the patent: **22.03.2023**
(21) Application number: 17880442.3
(22) Date of filing: 04.12.2017
(51) Int. Cl.: G01N 33/18, B01D 19/00, G01N 1/10

(54) **SHIP-MOUNTED WATER QUALITY ANALYSIS APPARATUS AND SHIP-MOUNTED DEFOAMING DEVICE**
SCHIFFSMONTIERTE VORRICHTUNG ZUR WASSERQUALITÄTSANALYSE UND SCHIFFSMONTIERTE ENTSCHÄUMUNGSVORRICHTUNG
APPAREIL D'ANALYSE DE LA QUALITÉ DE L'EAU ET DISPOSITIF DE DÉMOUSSAGE MONTÉS SUR UN NAVIRE

(30) Priority: 15.12.2016 JP 2016243811
(43) Date of publication of application: 09.10.2019
(73) Proprietor: Horiba Advanced Techno Co., Ltd., Kyoto-shi, Kyoto 601-8306 (JP)
(72) Inventor: HASHIMA, Yuta, Kyoto-shi Kyoto 601-8306 (JP); TOMIOKA, Kiichiro, Kyoto-shi Kyoto 601-8306 (JP); KAWANO, Tadashi, Kyoto-shi Kyoto 601-8306 (JP)
(74) Representative: Isarpatent
(86) International application number: PCT/JP2017/043478
(87) International publication number: WO 2018/110345

(56) References cited:
- EP-A2- 1 445 611
- JP-A- S5 578 597
- JP-A- H08 136 526
- JP-A- 2003 075 344
- JP-A- 2009 031 173
- JP-A- 2009 031 173
- JP-A- 2010 281 679
- JP-A- 2017 136 554
- JP-Y1- S4 829 198
- US-A1- 2003 047 067

## Description

### Technical Field

The present invention relates to a debubbler for installing on a ship.

### Background Art

In recent years, exhaust gas regulations for ships have been being strengthened, and reductions in the discharge amounts of SOx, NOx, and the like discharged from an internal combustion engine of a ship have been being required. For this reason, among ships, there are ones configured to be able to clean exhaust gas through a scrubber in order to clean the exhaust gas and then discharge it.

In the scrubber, the exhaust gas discharged from an internal combustion engine is flowed, and also pumped seawater is sprayed as microdroplets. Further, particles containing Sox, NOx, and the like contained in the exhaust gas are separated and collected in the sprayed droplets, and therefore the exhaust gas passing through the scrubber is cleaned.

In addition, the seawater containing SOx and NOx discharged from the scrubber has to be subjected to wastewater treatment so as to satisfy predetermined environmental standards, and then returned to the sea again. For this reason, whether or not the wastewater treatment is adequately performed on the ship has to be constantly monitored by a water quality analysis apparatus.

Meanwhile, if there are bubbles in sample liquid when performing water quality analysis, the bubbles blocks the sample liquid from contacting with, for example, a sensor, or causes a measurement error of an analysis device due to changing absorbance, or the like. For this reason, as in a water quality analysis apparatus disclosed in Patent Literature 1, bubbles included in sample liquid are removed by a debubbler and then analysis is performed by an analysis device.

However, a ship may pitch and roll heavily in various directions at sea, and therefore even if a water quality analysis apparatus provided with a debubbler as described above is used, sample liquid and air are mixed to produce new bubbles in the sample liquid on the way to an analysis device, and a measurement error cannot be sufficiently reduced in some cases.

### Citation List

### Patent Literature

### Patent Literature 1

Japanese Unexamined Patent Publication JP-A2000-126507. US 2003/047067 A1 describes a method with the goal of increasing the effectiveness of a standard debubbler, accomplished through operating an ultrasonic bubble eliminator at a constant output voltage near the parallel resonant frequency of the eliminator's transducer. EP 1 445 611 A2 describes an apparatus for the continuous measurement of the quality of the water in a waterway, which includes a bubble removal chamber before the measurement sensor which removes bubbles contained in the water by creating a whirlpool in the bubble removal chamber. JP 2009/031173 A describes a particle measurement device for measuring micro particles in a liquid. The device comprises a pressure vessel with a cell made of quartz glass, an ultrasonic oscillator, a pump for pressure reduction; a particle counter for measuring micro particles in a deaerated medium, and a syringe pump for sucking the medium. By ultrasonic vibration propagating to the medium in the cell under pressure reduction, air bubbles are removed and the particles in liquid are measured.

### Summary of Invention

### Technical Problem

The present invention has been made in consideration of the problem as described above, and intends to provide a debbubler for installing on a ship capable of supplying, to an analysis device, sample liquid from which bubbles are sufficiently removed, and reducing a measurement error to continuously monitor accurate water quality even on a ship.

### Solution to Problem

That is, a debubbler for installing on a ship according to the present invention is defined in the appended claims. The following illustrative examples not forming part of the invention include a ship-installed water quality analysis apparatus comprising a debubbler that is installed in a ship and removes bubbles from sample liquid; and at least one analysis device that is provided downstream of the debubbler and analyzes the water quality of the sample liquid from which the bubbles are removed by the debubbler, in which the debubbler includes: a housing container in which the sample liquid is housed; a liquid introduction port for introducing the sample liquid into the housing container; an ultrasonic transducer that applies ultrasonic vibration to the sample liquid in the housing container; a bubble discharge port for discharging bubbles produced in the sample liquid by the ultrasonic vibration to the outside from the upper part of the housing container; and a liquid lead-out port for leading the sample liquid from which the bubbles are removed in the housing container to the outside to supply it to the analysis device, and an inside of the housing container is configured to be in a state of being filled with the sample liquid.

Also, the ship-installed water quality analysis apparatus according to an example includes: a debubbler that is installed in a ship and removes bubbles from sample liquid; and at least one analysis device that is provided downstream of the debubbler and analyzes water quality of the sample liquid from which the bubbles are removed by the debubbler, in which the debubbler includes: a housing container in which the sample liquid is housed; a liquid introduction port for introducing the sample liquid into the housing container; an ultrasonic transducer that applies ultrasonic vibration to the sample liquid in the housing container; a bubble discharge port for discharging bubbles produced in the sample liquid by the ultrasonic vibration to an outside from an upper part of the housing container; and a liquid lead-out port for leading the sample liquid from which the bubbles are removed in the housing container to the outside to supply it to the analysis device, and is configured to discharge part of the sample liquid as well from the bubble discharge port together with the bubbles.

In such configurations, even when the ship pitches and rolls, air causing the production of bubbles hardly exists in the housing container, and therefore new bubbles are hardly produced in the sample liquid inside the housing container. Also, bubbles originally included in the sample liquid are increased in diameter by the ultrasonic vibration to rise in the housing container, and discharged outside from the bubble discharge port, and therefore the sample liquid led out from the liquid lead-out port hardly includes bubbles to make it possible to significantly reduce a measurement error of the analysis device. For this reason, accurate continuous monitoring of water quality becomes possible even on a ship.

In order to make it more easy to separate bubbles by depressurizing the sample liquid to be introduced into the housing container, as well as make it difficult to produce bubbles by bring the sample liquid to be supplied to the analysis device into a pressurized state, it is only necessary to further include: a first valve that is provided in a bubble discharge line connected to the bubble discharge port; and a second valve that is provided in a liquid lead-out line connected to the liquid lead-out port. For example, it is only necessary that, before factory shipment, the opening level of the first valve is set so that the bubbles and the sample liquid are discharged in a mixed state from the bubble discharge port, and the opening level of the second valve is set to a predetermined opening level and fixed so as to make the flow velocity of the sample liquid led out from the liquid lead-out port lower than the flow velocity of the sample liquid flowing into the housing container. In addition, the first valve and the second valve may be adapted to be able to appropriately change the opening levels thereof on the ship, or may be ones whose opening levels are fixed at opening levels fixed in advance before the factory shipment.

In order to make it possible to, even when a pressure variation in and a flow rate variation in the sample liquid occur on the supply side from which the sample liquid is supplied to the debubbler, keep constant a state of the sample liquid from which the bubbles are removed and that is to be supplied to the analysis device and accurately achieve continuous monitoring, it is only necessary to further include a regulator that is provided in a liquid introduction line connected to the liquid introduction port, and regulates the pressure of the sample liquid to be introduced into the housing container to a predetermined pressure. In such a configuration, a pressure variation in the sample liquid flowing into the debubbler can be reduced, and the inflow sample liquid can be limited so as to have an appropriate flow rate.

In order to make it possible for an operator to easily check whether or not the inside of the housing container is filled up with a sufficient amount of the sample liquid, and a substantially filled-up state is achieved, it is only necessary that at least part of a bubble discharge line connected to the bubble discharge port is formed of a transparent pipe. In such a configuration, the operator checks that the sample liquid flows through the bubble discharge port, and thereby even when the sample liquid is in a state of continuously flowing in/out, it is possible to check that the inside of the housing container is filled with the sample liquid and filled up. Accordingly, the operator can easily determine that a state where new bubbles due to the pitch and roll of the ship are hardly produced lies.

In order to make it possible to prevent new bubbles from being produced in the sample liquid also in a portion other than the housing container to cause a measurement error, it is only necessary that the analysis device includes a flow cell through which the sample liquid from which the bubbles are removed and that is supplied from the lead-out part flows, and is configured so that the inside of the housing container and the inside of the flow cell are brought into a state of being substantially filled up with the sample liquid.

In order to make it possible to, even when bubbles flows into the flow cell, prevent the bubbles from being kept accumulated in a position causing a measurement error, such as a sensing part of the analysis device, it is only necessary that the analysis device has a sensor surface that is arranged so as to contact with the sample liquid in the flow cell, and the sensor surface is provided tilted with respect to the horizontal plane in a natural state. In such a configuration, the bubbles flowing into the flow cell can be naturally moved upward along the sensor surface.

In order to increase a velocity at which the sample liquid flows along the sensor surface, and even when bubbles are present in the sample liquid, immediately move it from the sensor surface to prevent causing a measurement error, it is only necessary that the flow cell is provided opposite to the sensor surface, and further includes an introduction port for introducing the sample liquid toward the sensor surface, and the introduction port has a narrowed structure.

In order to make it possible to supply, to an analysis device, sample liquid, from which bubbles have been sufficiently removed and achieve accurate water quality analysis even on a ship, it is only necessary to use a ship-installed debubbler including: a housing container that is installed in a ship, houses sample liquid, and extends in an up-down direction; a liquid introduction port for introducing the sample liquid into the housing container; an ultrasonic transducer that applies ultrasonic vibration to the sample liquid in the housing container; a bubble discharge port for discharging bubbles produced in the sample liquid by the ultrasonic vibration to an outside from an upper part of the housing container; and a liquid lead-out port for leading the sample liquid from which the bubbles are removed to the outside from the lower part of the housing container to supply it to an analysis device, in which the inside of the housing container is configured to be in a state of being filled with the sample liquid.

What are suitable for removing bubbles from sample liquid on a ship can include a ship-installed debubbler including: a housing container in which the sample liquid is housed; a liquid introduction port for introducing the sample liquid into the housing container; an ultrasonic transducer that applies ultrasonic vibration to the sample liquid in the housing container; a bubble discharge port for discharging bubbles produced in the sample liquid by the ultrasonic vibration to an outside from an upper part of the housing container; and a liquid lead-out port for leading the sample liquid from which the bubbles are removed in the housing container to the outside to supply it to an analysis device, and is configured to discharge part of the sample liquid as well from the bubble discharge port together with the bubbles.

### Advantageous Effects of Invention

The ship-installed water quality analysis apparatus according to the present invention can make it difficult to produce new bubbles in the debubbler to constantly perform accurate monitoring even when a ship pitches and rolls.

### Brief Description of Drawings

[Fig. 1]

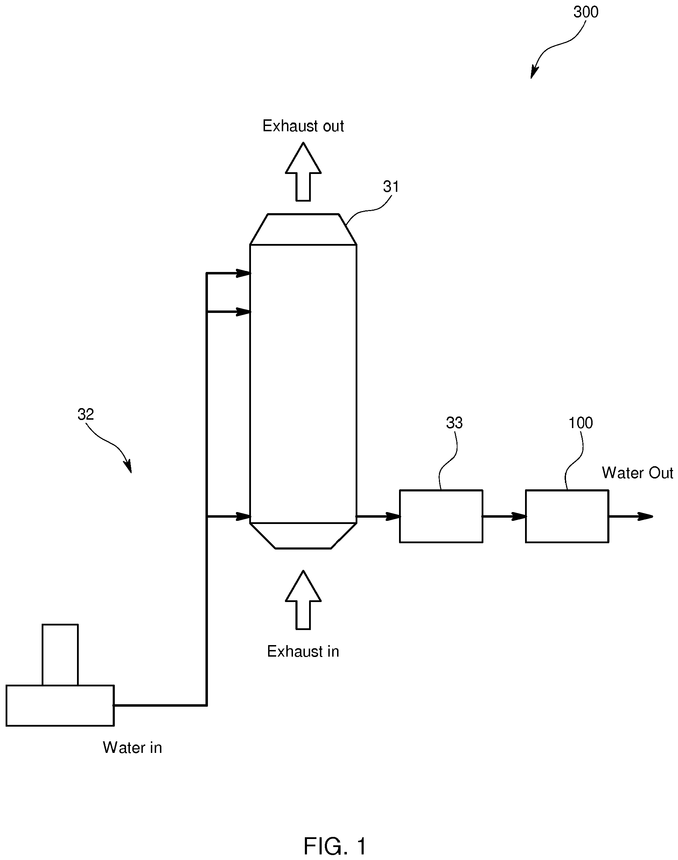

   Fig. 1 is a schematic diagram illustrating the outline of an exhaust gas cleaning system in which a ship-installed water quality analysis apparatus according to one illustrative example is used.
[Fig. 2]

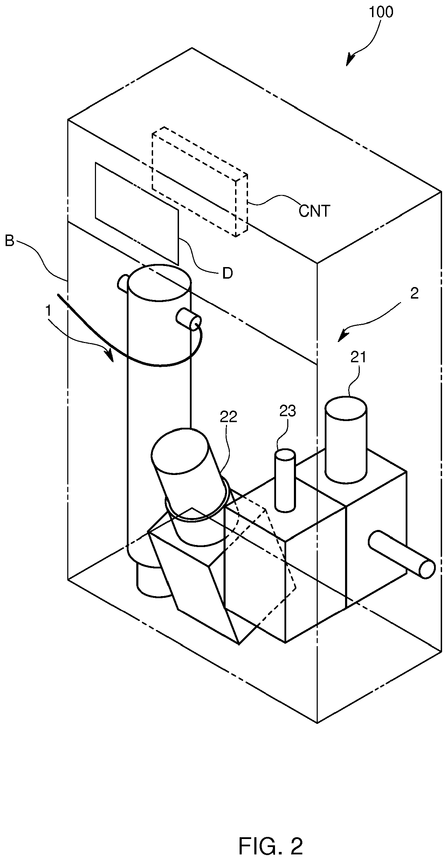

   Fig. 2 is a schematic perspective view of the ship-installed water quality analysis apparatus in the same example.
[Fig. 3]

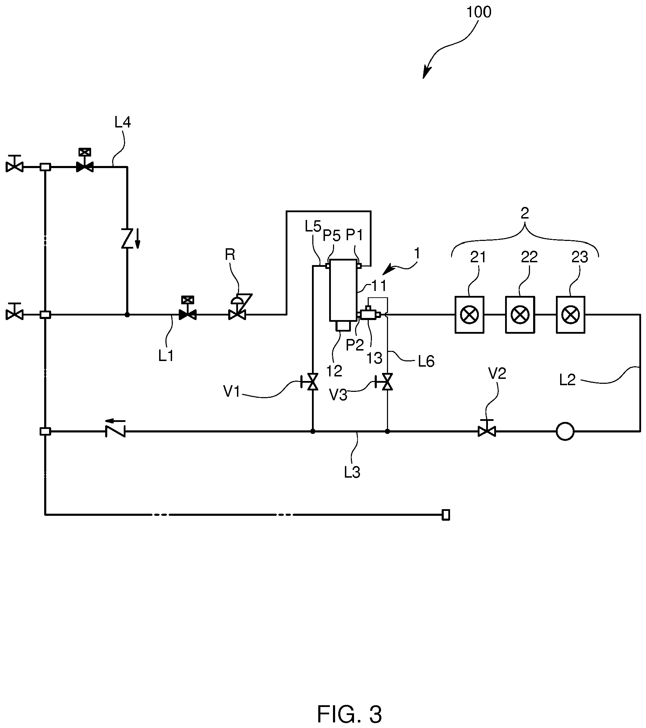

   Fig. 3 is a schematic flow diagram of the ship-installed water quality analysis apparatus in the same example.
[Fig. 4]

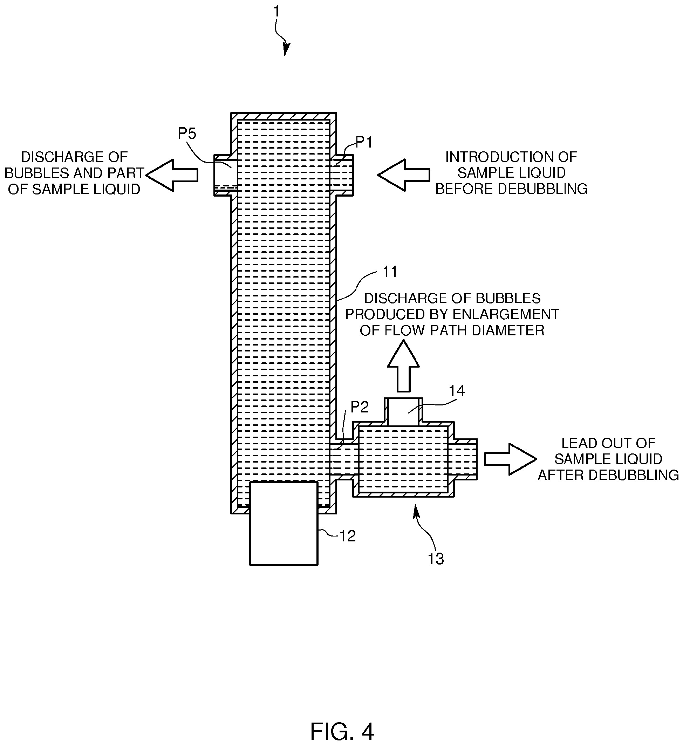

   Fig. 4 is a schematic partial cross-sectional view illustrating the internal structure of a debubbler in the same example.
[Fig. 5]

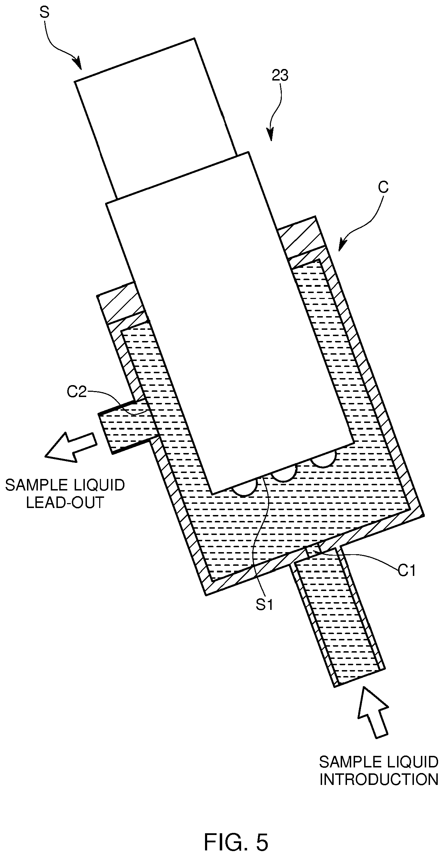

   Fig. 5 is a schematic partial cross-sectional view illustrating the internal structure of an analysis device in the same example.
[Fig. 6]

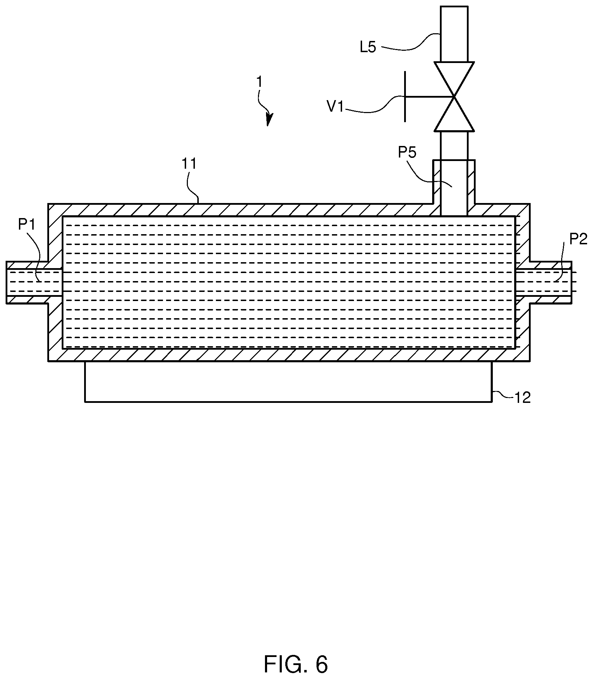

   Fig. 6 is a schematic cross-sectional view illustrating the configuration of a debubbler of a ship-installed water quality analysis apparatus according to another illustrative example.

### Reference Signs List

100 Ship-installed water quality analysis apparatus
1 Debubbler
11 Housing container
12 Ultrasonic transducer
P1 Introduction port
P2 Lead-out port
P5 Bubble discharge port
2 Analysis devices
21 Turbidity meter
22 PAH meter
23 pH meter
C Flow cell
C1 Introduction port
C2 Lead-out port
C3 Tilted surface
V1 First valve
V2 Second valve

### Description of Embodiments

A ship-installed water quality analysis apparatus 100 according illustrative examples not forming part of the invention will be described with reference to Fig. 1 to Fig. 5. The ship-installed water quality analysis apparatus 100 of the present example is one used to constantly monitor the water quality of wastewater discharged from an exhaust gas cleaning system 300 on a ship, and check whether or not wastewater treatment in the exhaust gas cleaning system 300 operates as regulated.

As illustrated in Fig. 1, the exhaust gas cleaning system 300 is one that cleans SOx and NOx discharged from an internal combustion engine of a ship to produce exhaust gas satisfying various regulation values, and discharges it. More specifically, the exhaust gas cleaning system 300 includes: a scrubber 31; a cleaning liquid supply mechanism 32 that, as cleaning liquid, supplies seawater collected from outside the ship to the scrubber 31; a wastewater treatment mechanism 33 that cleans wastewater discharged from the scrubber 31; and the ship-installed water quality analysis apparatus 100 that monitors the water quality of the wastewater cleaned by the wastewater treatment mechanism 33.

The scrubber 31 is a substantially cylindrical one into which exhaust gas discharged from the internal combustion engine is introduced from below and inside which as the cleaning liquid, droplet-shaped seawater or water is sprayed from the cleaning liquid supply mechanism 32. Particles containing SOx and NOx in the exhaust gas are aggregated in the droplet-shaped seawater and led out to the wastewater treatment mechanism 33 from the lower part of the scrubber 31. On the other hand, the cleaned exhaust gas is discharged to the air from above the scrubber 31.

The wastewater treatment mechanism 33 is one that has a function of, for example, centrifugally separating the wastewater to remove the aggregated particles component contained in the wastewater, as well as adjusting the pH of the wastewater.

The ship-installed water quality analysis apparatus 100 is one that is introduced as sample liquid with the cleaned wastewater discharged from the wastewater treatment mechanism 33, and continuously monitors turbidity, PAH, and pH as the water quality of it. As illustrated in a perspective view of Fig. 2, the ship-installed water quality analysis apparatus 100 is such that respective devices are housed in a casing B. In the casing B, a debubbler 1 that removes bubbles from the sample liquid and three types of analysis devices 2 that analyze the water quality of the sample liquid from which the bubbles are removed are housed. In addition, the ship-installed water quality analysis apparatus includes: a control part CNT that controls at least below-described respective valves; and a display D on which control states of the respective devices, measurement results by the analysis devices 2, and the like are displayed. As illustrated in a flow diagram of Fig. 3, the ship-installed water quality analysis apparatus 100 includes as main lines related to the water quality analysis of the sample liquid: a liquid introduction line L1 for introducing the sample liquid from the wastewater treatment mechanism 33 to the debubbler 1; a liquid lead-out line L2 for supplying the sample liquid from which the bubbles are removed from the debubbler 1 to the multiple analysis devices 2; and a wastewater line L3 through which bubbles resulting from debubbling by the debubbler 1 or the sample liquid is discharged to outside the apparatus or outside the ship. Further, the ship-installed water quality analysis apparatus 2 includes: a zero water supply line L4 that is provided in parallel with the liquid introduction line L1 to supply zero water used for adjustment to the debubbler 1 and the analysis devices 2; and a bubble discharge line L5 that connects between the upper part of the debubbler 1 and the wastewater line L3 and through which the bubbles resulting from the debubbling are flowed from the debubbler 1 to the wastewater line L3. In addition, in the present embodiment, a flow path from the debubbler 1 to the below-described second valve V2 is defined as the introduction line L1, and a flow path on the downstream side of the second valve V2 is defined as the wastewater line L3.

The details of the respective parts will be described.

As illustrated in Fig. 2 and Fig. 4, the debubbler 1 includes: a housing container 11 that houses the sample liquid introduced from the wastewater treatment mechanism 33 and extends in an up-down direction; a liquid introduction port P1 for introducing the sample liquid into the housing container 11; an ultrasonic transducer 12 that is provided in the lower part of the housing container 11 and applies ultrasonic vibration to the sample liquid in the housing container 11; a bubble discharge port P5 for discharging the bubbles produced in the sample liquid by the ultrasonic vibration to the outside from the upper part of the housing container 11; and a liquid lead-out port P2 for leading the sample liquid from which the bubbles are removed to the outside from the lower part of the housing container 11 to supply it to the analysis devices 2.

The housing container 11 is a substantially hollow cylindrical one, and in the upper side surface thereof, the liquid introduction port P1 connected to the liquid introduction line L1 is formed. Also, in the housing container 11, the bubble discharge port P5 is formed in a position above the liquid introduction port P1, and connected with the bubble discharge line L5. The liquid lead-out port P2 is provided in the side surface near the bottom surface of the housing container 11, and connected to the liquid lead-out line L2. The ultrasonic transducer 12 is attached to the bottom surface of the housing container 11 in such a manner as to contact with the sample liquid inside, and adapted to allow the ultrasonic vibration to produce the bubbles in a position higher than the liquid lead-out port P2 in the housing container 11 and in a position lower than the bubble discharge port P5 and the liquid introduction port P1.

In the liquid introduction line L1, a regulator R that regulates the pressure of the sample liquid supplied from the wastewater treatment mechanism 33 to a predetermined pressure is provided, and a constant pressure of the sample liquid is adapted to be introduced into the housing container 11. More specifically, a pressure variation in and a flow rate variation in the sample liquid due to the centrifugal separation in the wastewater treatment mechanism 33 can be adapted to be reduced by the regulator R to supply a constant flow rate of the sample liquid to the debubbler 1 and the respective analysis devices 2.

Also, in the bubble discharge line L5, a first valve V1 is provided, and in the liquid lead-out line L2, the second valve V2 is provided. The opening level of the first valve V1 has been set and the opening level has been fixed before factory shipment so that not only the bubbles are discharged to the bubble discharge line L5 but part of the sample liquid in the housing container 11 is discharged to the bubble discharge line L5 and the pressure of the sample liquid in the housing container 11 is made lower than at the time of inflow. Also, the opening level of the second valve V2 has been set and the opening level has been fixed before the factory shipment so as to make the flow velocity of the sample liquid led out from the liquid lead-out port P2 lower than the flow velocity of the sample liquid flowing into the housing container 1 to bring the sample liquid in the liquid lead-out line L2 and in the respective analysis devices 2 into a pressurized state. Further, the pressure ratio among the liquid introduction line L1, the bubble discharge line L5, and the liquid lead-out line L2 is adapted to be kept at a predetermined value by the first valve V1 and the second valve V2, and the sample liquid to be introduced is adapted to be distributed between the bubble discharge line L5 and the liquid introduction line L1 at a predetermined ratio. In addition, part of the sample liquid is adapted to be discharged also from the bubble discharge line L5, and thereby as illustrated in a cross-sectional view of Fig. 4, the inside of the housing contained 11 is adapted to be constantly substantially filled with the sample liquid. Note that "substantially filled" refers to a state where the sample liquid is filed up to the inner upper surface of the housing container 11 in such a manner as to contact with it. In other words, even when the ship pitches and rolls, the inside of the housing container 11 is kept in a state where a wave front is hardly produced on the upper surface of the sample liquid.

Also, at least part of the bubble discharge line L5 is formed of a transparent pipe to enable an operator to visually check the flow of the sample liquid discharged from inside the housing container 11.

In addition, at the outlet of the liquid lead-out port P2 is connected with a flow path diameter enlarged part 13 whose flow path diameter is larger than the liquid lead-out port P2, and the flow velocity of the sample liquid led out of the housing container 11 is adapted to be reduced to further produce bubbles remaining in the sample liquid. An upper opening 14 provided in the flow path diameter enlarged part 13 is connected to the wastewater line L3 via an enlarged part bubble discharge line L6, and in the enlarged part bubble discharge line L6, a third valve V3 is provided. The opening level of the third valve V3 has been set before the factory shipment so that only bubbles produced inside the flow path diameter enlarged part 13, or the bubbles and part of the sample liquid are taken outside via the upper opening 14 and discharged to the wastewater line L3.

Next, the respective analysis devices 2 will be described with reference to Fig. 2 and Fig. 5.

In the liquid lead-out line L2, a turbidity meter 21, PAH meter 22 (polycyclic aromatic hydrocarbon meter), and pH meter 23 are provided sequentially from the upstream side as the analysis devices 2. Each of the analysis devices 2 includes a substantially hollow rectangular parallelepiped flow cell C to which the sample liquid from which the bubbles are removed by the debubbler 1 is supplied and flows. Each flow cell C is of a substantially rectangular parallelepiped shape, and as illustrated in a perspective view of Fig. 2, in the casing, the flow cell of the PAH meter 22 is provided tilted with respect to the horizontal plane in a natural state. Note that the turbidity meter 21 and the pH meter 23 are provided such that axial directions thereof coincide with the vertical direction in the natural state. The turbidity meter 21 and the PAH meter 22 are adapted such that on the basis of the absorbance of the sample liquid at a predetermined wavelength, the values thereof are continuously measured. Also, the pH meter 23 is adapted such that on the basis of a potential difference generated by a glass electrode method, pH is continuously measured.

Next, the details of the PAH meter 22 will be described with reference to a cross-sectional view of Fig. 5. The flow cell C of the PAH meter 22 is configured such that the sample liquid is introduced from an introduction port C1 in the bottom surface and led outside from a lead-out port C2 in the central part of a side surface thereof. A sensor surface S1 on which a light source and a detector constituting a sensor S portion of the PAH meter 22 are exposed to the outside of a housing is arranged in the internal central part of the flow cell C, and also tilted at a predetermined angle with respective to the horizontal plane in the natural state where the ship is stationary. A lower surface opposite to the sensor surface S1 is formed with the introduction port C1 so as to introduce the sample liquid toward a tilted surface C3. The introduction port C1 is made partially narrower than the diameter of the liquid lead-out line L2 to form a narrowed structure, and adapted to increase the flow velocity of the sample liquid introduced toward the sensor surface S1 in the flow cell C to blow it to the sensor surface S1. By forming the flow of the sample liquid in the flow cell C in this manner, even if bubbles are included in the sample liquid, the bubbles are adapted to move obliquely upward along the sensor surface S1 and be prevented from being kept accumulated on the sensor surface S1.

In the present example, as illustrated in Fig. 5, not only the housing container 11, but also the inside of the flow cell C of the PAH meter 22 is configured to be kept in a state of being substantially filled up with the sample liquid. In addition, the other flow cells C of the turbidity meter 21 and the pH meter 23 are also adapted so that the insides thereof are kept in the state of being substantially filled up with the sample liquid. Further, the inside of the liquid lead-out line L2 is also adapted to be kept filled up with the liquid, and the entire flow path from the debubbler 1 to the pH meter 23 as the last analysis device 2 is adapted to be kept in the state of being substantially filled up with the liquid. Note that "the inside of a flow cell C is substantially filled up with the sample liquid" means that, for example, the inside of the flow cell C is filled with the sample liquid, and the sample liquid reaches the inner upper surface of the flow cell C or the highest position of the inner upper surface in the natural state.

In the ship-installed water quality analysis apparatus 100 configured as described above, since the flow path forming a measurement system from the debubbler 1 to the pH meter 23 as the last analysis device 2 is in the state of being filled up with the sample liquid, even when the ship pitches and rolls, the sample liquid can be prevented from being mixed with air to produce new bubbles. Accordingly, a state where the bubbles in the sample liquid are removed by the debubbler 1 is constantly kept, and a measurement error due to bubbles can be prevented from occurring in each of the analysis devices 2. Also, since the sample liquid is adapted to be blown toward the tilted surface C3 in the flow cell C, even if a small number of bubbles are produced after the debubbling, the bubbles are moved upward along the tilted surface C3 and prevented from being kept accumulated on the sensor.

Accordingly, even in environments where the pitch and roll are large as in ships and a stable measurement environment cannot be obtained, the effect of bubbles can be eliminated to achieve correct measurement.

Other illustrative examples not forming part of the invention will be described.

The housing container 11 of the debubbler 1 is not limited to one extending in the up-down direction, but like a housing container 1 illustrated in Fig. 6, may be one extending in the horizontal direction. More specifically, the respective end surfaces of the housing container 1 provided in the horizontal direction are respectively provided with a liquid introduction port P1 and a liquid lead-out port P2, and the upper side among the side surfaces of the housing container 1 is provided with a bubble discharge port P5. Further, the lower side among the side surfaces of the housing container 1 is provided with, for example, an ultrasonic transducer 12 having the same level of length as the horizontal length dimension of the housing container 1, and internal sample liquid is adapted to be applied with ultrasonic vibration from the side surface side of the housing container 1. In this embodiment as well, the inside of the housing container 1 is adapted to be in a state of being filled with the sample liquid in a state where the sample liquid flows into/out of the inside of the housing container 1. Specifically, from the bubble discharge port P5, not only bubbles produced by the ultrasonic vibration, but also part of the sample liquid in the housing container 1 are adapted to be discharged. That is, as illustrated, in the state where the sample liquid continuously flows in/out, the sample liquid is configured to keep maintaining a state of contacting with all the inner wall surfaces excluding the wall surfaces provided with the respective ports of the housing container 1 substantially without any gap.

A position where the ultrasonic transducer is provided in the debubbler is not limited to the bottom surface of the housing container, and it may be provided on a side surface or the upper surface. Also, the ultrasonic transducer does not directly contact with the inside of the housing container, but may be adapted to indirectly ultrasonically vibrate the sample liquid by vibrating a wall surface of the housing container. Further, the housing container itself may be formed of transparent resin to enable whether or not in the state where the sample liquid continuously flows into/out of the inside of the housing container, it is wholly filled with the sample liquid to be visually checked.

The ship-installed water quality analysis apparatus according to the present invention may be used not only for monitoring wastewater in the cleaning system for SOx and NOx but also for other applications. In addition, a place where the ship-installed water quality analysis apparatus is provided only has to be appropriately selected depending on sample liquid desired to be monitored.

The installation number of analysis devices may be one or two, or may be four or more. In addition, water quality analysis targets are not limited to turbidity, PAH, and pH, and analysis devices may measure other properties. Further, the order of providing the respective analysis devices are not limited to that described in the above-described embodiment. One whose sensor surface provided with a sensing part, a light source, and a detector of a sensor is tilted with respect to the horizontal plane in the natural state is not limited only to the PAH meter, and the pH meter and the turbidity meter may also employ the same configuration. Further, the flow cell of the rectangular parallelepiped shape is not tilted in the natural state but may be provided extending in the up-down direction, and only the sensor itself may be provided tilted.

In order to make it possible to check whether or not the sample liquid is discharged from the housing container to the bubble discharge line and the inside of the housing container is filled up with the sample liquid, the bubble discharge line may be provided with a pressure gauge to make it possible for an operator to make a determination on the basis of whether or not the pressure gauge indicates a predetermined pressure value.

Only the analysis devices may be housed in the casing, and the debubbler may be installed outside the casing. Also, as long as at least the inside of the housing container is filled up with the liquid, new bubble production due to the pitch and roll of the ship can be significantly reduced as compared with before, and a measurement error can also be reduced.

### Industrial Applicability

The present invention can provide the ship-installed water quality analysis apparatus that makes it difficult to produce new bubbles in the debubbler even when a ship pitches and rolls and is capable of constantly performing accurate monitoring.

## Claims

1. A debubbler (1) for installing on a ship, comprising:
a housing container (11) that is installed in a ship and in which sample liquid is housed;
a liquid introduction port (P1) for introducing the sample liquid into the housing container (11) through a liquid introduction line (L1);
an ultrasonic transducer (12) that applies ultrasonic vibration to the sample liquid in the housing container (11);
a bubble discharge port (P5) for discharging bubbles produced in the sample liquid by the ultrasonic vibration to an outside from an upper part of the housing container (11);
a liquid lead-out port (P2) for leading the sample liquid from which the bubbles are removed in the housing container (11) to an analysis device (2) configured to analyze water quality of the sample liquid,
a first valve (V1) that is provided in a bubble discharge line (L5) connected to the bubble discharge port (P5); and
a second valve (V2) that is provided in a liquid lead-out line (L2) connected to the liquid lead-out port (P2), wherein
the first valve (V1) and the second valve (V2) have opening levels such that of the sample liquid is adapted to be discharged also from the bubble discharge port together with the bubbles, thereby filling an inside of the housing container (11) with the sample liquid.

2. The debubbler (1) according to claim 1, further comprising a regulator (R) that is provided in the liquid introduction line (L1) connected to the liquid introduction port (P1), and regulates pressure of the sample liquid to be introduced into the housing container (11) to a predetermined pressure.

3. The debubbler (1) according to any of claims 1 or 2, wherein
at least part of a bubble discharge line (L5) connected to the bubble discharge port (P5) is formed of a transparent pipe.

4. A water quality analysis apparatus (100) for installing on a ship, comprising
the debubbler (1) according to any of claims 1 to 3, and
at least one analysis device (2) that is provided downstream of the debubbler (1) and analyzes water quality of the sample liquid from which the bubbles are removed by the debubbler (1); wherein
the analysis device (2) comprises a flow cell (C) through which the sample liquid from which the bubbles are removed and that is supplied from the lead-out port (P2) flows, and
is configured so that the inside of the housing container (11) and an inside of the flow cell (C) are brought into a state of being filled with the sample liquid.

5. The water quality analysis apparatus (100) according to claim 4, wherein
the analysis device (2) has a sensor surface (S1) that is arranged so as to contact with the sample liquid in the flow cell (C), and
the sensor surface (S1) is provided tilted with respect to a horizontal plane in a natural state.

6. The water quality analysis apparatus (100) according to claim 5, wherein
the flow cell (C) is provided opposite to the sensor surface (S1), and further comprises an introduction port (C1) for introducing the sample liquid toward the sensor surface (S1), and the introduction port (C1) has a narrowed structure.

## Patentansprüche

1. Vorrichtung zum Entfernen von Blasen (1) zum Installieren auf einem Schiff, umfassend:
einen Aufnahmebehälter (11), der in einem Schiff installiert ist und in dem Probenflüssigkeit aufgenommen ist;
einen Flüssigkeitseinleitungsport (P1) zum Einleiten der Probenflüssigkeit in den Aufnahmebehälter (11) durch eine Flüssigkeitszuleitung (11);
einen Ultraschallwandler (12), der Ultraschallschwingungen an die Probenflüssigkeit in dem Aufnahmebehälter (11) anlegt;
einen Blasenauslassport (P5) zum Auslassen von Blasen, die in der Probenflüssigkeit durch die Ultraschallschwingungen erzeugt werden, von einem oberen Teil des Aufnahmebehälters (11) nach draußen;
einen Flüssigkeitsabflussport (P2) zum Leiten der Probenflüssigkeit, aus der die Blasen in dem Aufnahmebehälter (11) entfernt wurden, zu einer Analysevorrichtung (2), die dafür ausgebildet ist, eine Wasserqualität der Probenflüssigkeit zu analysieren,
ein erstes Ventil (V1), das in einer Blasenauslassleitung (L5) angeordnet ist, die mit dem Blasenauslassport (P5) verbunden ist; und
ein zweites Ventil (V2), das in einer Flüssigkeitsabflussleitung (L2) angeordnet ist, die mit dem Flüssigkeitsabflussport (P2) verbunden ist, wobei das erste Ventil (V1) und das zweite Ventil (V2) solche Öffnungsgrade aufweisen, dass ein Teil der Probenflüssigkeit zusammen mit den Blasen ebenfalls aus dem Blasenauslassport ausgelassen werden kann, wodurch ein Innenraum des Aufnahmebehälters (11) mit der Probenflüssigkeit gefüllt wird.

2. Vorrichtung zum Entfernen von Blasen (1) nach Anspruch 1, des Weiteren umfassend:
einen Regler (R), der in der Flüssigkeitszuleitung (11) angeordnet ist, die mit dem Flüssigkeitseinleitungsport (P1) verbunden ist und einen Druck der in den Aufnahmebehälter (11) einzuleitenden Probenflüssigkeit auf einen zuvor festgelegten Druck regelt.

3. Vorrichtung zum Entfernen von Blasen (1) nach einem der Ansprüche 1 und 2, wobei
mindestens ein Teil einer mit dem Blasenauslassport (P5) verbundenen Blasenauslassleitung (L5) von einem transparenten Rohr gebildet ist.

4. Wasserqualitätsanalysevorrichtung (100) zum Installieren auf einem Schiff, umfassend:
die Vorrichtung zum Entfernen von Blasen (1) nach einem der Ansprüche 1 bis 3, und
mindestens eine Analysevorrichtung (2), die der Vorrichtung zum Entfernen von Blasen (1) nachgeschaltet ist und die Wasserqualität der Probenflüssigkeit analysiert, aus der die Blasen durch die Vorrichtung zum Entfernen von Blasen (1) entfernt wurden; wobei
die Analysevorrichtung (2) eine Durchflusszelle (C) umfasst, durch die die Probenflüssigkeit strömt, aus der die Blasen entfernt wurden und die von dem Abflussport (P2) zugeführt wird, und
so ausgebildet ist, dass der Innenraum des Aufnahmebehälters (11) und ein Innenraum der Durchflusszelle (C) in einen Zustand gebracht werden, in dem sie mit der Probenflüssigkeit gefüllt sind.

5. Wasserqualitätsanalysevorrichtung (100) nach Anspruch 4, wobei
die Analysevorrichtung (2) eine Sensorfläche (S1) aufweist, die so angeordnet ist, dass sie mit der Probenflüssigkeit in der Durchflusszelle (C) in Kontakt kommt, und
die Sensorfläche (S1) so bereitgestellt ist, dass sie in einem natürlichen Zustand relativ zu einer horizontalen Ebene geneigt ist.

6. Wasserqualitätsanalysevorrichtung (100) nach Anspruch 5, wobei
die Durchflusszelle (C) gegenüber der Sensoroberfläche (S1) angeordnet ist und des Weiteren ein Einleitungsport (C1) zum Einleiten der Probenflüssigkeit in Richtung der Sensoroberfläche (S1) umfasst und der Einleitungsport (C1) eine verengte Struktur aufweist.

## Revendications

1. Dispositif pour l'élimination des bulles (1) destiné à être installé sur un navire, comprenant :
un récipient de réception (11) qui est installé dans un navire et dans lequel un liquide d'échantillon est reçu ;
un orifice d'introduction de liquide (P1) pour introduire le liquide d'échantillon dans le récipient de réception (11) à travers une ligne d'introduction de liquide (L1) ;
un transducteur ultrasonore (12) qui applique une vibration ultrasonore au liquide échantillon dans le récipient de réception (11) ;
un orifice de décharge de bulles (P5) pour décharger des bulles produites dans le liquide échantillon par la vibration ultrasonore vers un extérieur à partir d'une partie supérieure du récipient de réception (11) ;
un orifice de guidage de sortie de liquide (P2) pour guider le liquide d'échantillon duquel les bulles sont retirées dans le récipient de réception (11) vers un dispositif d'analyse (2) configuré pour analyser la qualité de l'eau du liquide d'échantillon,
une première vanne (V1) qui est prévue dans une ligne de décharge de bulles (L5) raccordée à l'orifice de décharge de bulles (P5) ; et
une seconde vanne (V2) qui est prévue dans une ligne de guidage de sortie de liquide (L2) raccordée à l'orifice de guidage de sortie de liquide (P2), dans lequel
la première vanne (V1) et la seconde vanne (V2) ont des niveaux d'ouverture de telle sorte qu'une partie du liquide d'échantillon est adaptée pour être déchargée également de l'orifice de décharge de bulles conjointement avec les bulles, remplissant ainsi un intérieur du récipient de réception (11) avec le liquide d'échantillon.

2. Dispositif pour l'élimination des bulles (1) selon la revendication 1, comprenant en outre
un régulateur (R) qui est prévu dans la ligne d'introduction de liquide (L1) raccordée à l'orifice d'introduction de liquide (P1), et qui régule la pression du liquide échantillon à introduire dans le récipient de réception (11) à une pression prédéterminée.

3. Dispositif pour l'élimination des bulles (1) selon l'une quelconque des revendications 1 ou 2, dans lequel au moins une partie d'une ligne de décharge de bulles (L5) raccordée à l'orifice de décharge de bulles (P5) est formée d'un tuyau transparent.

4. Appareil d'analyse de la qualité de l'eau (100) destiné à être installé sur un navire, comprenant
le dispositif pour l'élimination des bulles (1) selon l'une quelconque des revendications 1 à 3, et
au moins un dispositif d'analyse (2) qui est prévu en aval du dispositif pour l'élimination des bulles (1) et analyse la qualité de l'eau de l'échantillon de liquide duquel les bulles sont éliminées par le dispositif pour l'élimination des bulles (1) ; dans lequel
le dispositif d'analyse (2) comprend une cuve à circulation (C) à travers laquelle le liquide d'échantillon duquel les bulles sont retirées et qui est fourni par l'orifice de guidage de sortie (P2) s'écoule, et
est configuré de telle sorte que l'intérieur du récipient de réception (11) et un intérieur de la cuve à circulation (C) sont amenés dans un état de remplissage avec le liquide d'échantillon.

5. Appareil d'analyse de la qualité de l'eau (100) selon la revendication 4 dans lequel
le dispositif d'analyse (2) a une surface de capteur (S1) qui est disposée de façon à être en contact avec le liquide d'échantillon dans la cuve à circulation (C), et
la surface de capteur (S1) est prévue inclinée par rapport à un plan horizontal dans un état naturel.

6. Appareil d'analyse de la qualité de l'eau (100) selon la revendication 5, dans lequel
la cuve à circulation (C) est prévue à l'opposé de la surface de capteur (S1), et comprend en outre un orifice d'introduction (C1) pour introduire le liquide échantillon vers la surface de capteur (S1), et l'orifice d'introduction (C1) a une structure rétrécie.
